# FASCICULE DE BREVET EUROPEEN

(11) **EP 1 586 376 B1**
(45) Date de publication et mention de la délivrance du brevet: **01.07.2009**
(21) Numéro de dépôt: 05290631.0
(22) Date de dépôt: 22.03.2005
(51) Int. Cl.: B01J 29/80, C07C 6/12, B01J 29/26, B01J 29/48, B01J 29/78, B01J 23/36

(54) **Catalyseur comprenant une zéolithe 10MR et une zéolithe 12MR et son utilisation en transalkylation d'hydrocarbures alkylaromatiques**
Katalysator enthaltend einen 10MR Zeolith und einen 12MR Zeolith und seine Verwendung zur Transalkylierung von alkylaromatischen Kohlenwasserstoffen
Catalyst comprising a 10MR zeolite and a 12MR zeolite and its use in the transalkylation of alkylaromatic hydrocarbons

(30) Priorité: 14.04.2004 FR 0403887
(43) Date de publication de la demande: 19.10.2005
(73) Titulaire: Institut Français du Pétrole, 92852 Rueil Malmaison Cedex (FR)
(72) Inventeur: Serra Alfaro, José Manuel, 46019 Valencia (ES); Corma, Avelino, 46022 Valence (ES); Guillon, Emmanuelle, 69390 Vernaison (FR)

(56) Documents cités:
- EP-A- 1 077 083
- WO-A-00/38834
- WO-A-03/099719
- WO-A-20/04046278
- FR-A- 2 790 001
- US-A- 5 055 176
- US-A- 5 759 950
- US-A- 5 800 698

## Description

### Domaine technique

La présente invention se rapporte à un catalyseur utilisable par exemple dans les réactions de transformation des hydrocarbures aromatiques. De manière plus précise, elle concerne un catalyseur de transalkylation d'hydrocarbures alkylaromatiques et de préférence de transalkylation du benzène ou du toluène et de composés aromatiques contenant au moins 9 atomes de carbone, pour produire des xylènes. La présente invention concerne aussi la préparation dudit catalyseur ainsi que son utilisation dans un procédé de transalkylation d'hydrocarbures alkylaromatiques.

### Art antérieur

De nombreux catalyseurs de dismutation et/ou de transalkylation ont déjà été décrits dans l'art antérieur, et sont à base de mordénite (US-A-3.506.731, US-A-4.151.120, US-A-4.180.693, US-A-4.210.770, US-A-3.281.483, US-A-3.780.121, ou US-A-3.629.351), ou bien aussi à base de zéolithe oméga (US-A-5.210.356, US-A-5.371.311).

Dans la demande de brevet EP-A1-0.731.071, l'utilisation d'un catalyseur à base de zéolithe mordénite et d'un métal (Re, Ni, Co, Mo, Cr et W) est décrit en transalkylation de coupes C9 aromatiques contenant un aromatique ayant au moins un groupement éthyle.

La demande de brevet FR-A-2 744 650 décrit l'utilisation d'un catalyseur composite de dismutation / transalkylation d'hydrocarbures alkylaromatiques à base de zéolithe de type structural Mordénite et de type structural Mazzite.

Le brevet US 5.942.651 décrit un système catalytique comprenant deux compositions catalytiques distinctes et séparées, l'une à base de zéolithe ayant un indice de contrainte compris entre 0.5 et 3 et de métal noble et la seconde à base de zéolithe ayant un indice de contrainte compris entre 3 et 12 sans métal ajouté. Le brevet US 5.905.051 divulgue un système catalytique comprenant une première composition catalytique à base de zéolithe Béta promue par un métal et une seconde composition catalytique à base de zéolithe ZSM-5 sur laquelle est imprégné un promoteur (S, P, Si). Les systèmes catalytiques divulgués dans ces documents sont utilisés dans un procédé pour la conversion de composés aromatiques comportant au moins 9 atomes de carbone par molécule.

Le document WO 2004/046278 divulgue un catalyseur de transalkylation comportant une zéolithe ZSM-5 (10 MR), une zéolithe mordénite (12 MR), de l'alumine et au moins un métal tel que le rhénium, molybdène, le tungstène, le vanadium, le chrome, le manganèse ou un métal du groupe VIII

Le document US 5.055.176 divulgue un catalyseur pour le craquage catalytique comportant au moins une zéolithe Y et au moins une zéolithe Ga-ZSM-5 ainsi qu'une matrice. La zéolithe RE-USY figurant dans l'exemple 1 est une zéolithe Y comprenant des terres rares.

Le document EP-A1-1.514.844 divulgue une zéolithe ITQ-22 éventuellement présente dans un catalyseur en association avec une zéolithe 12 MR ou une zéolithe 10 MR.

Le document US 5.759.950 divulgue un catalyseur d'isomérisation des alkylaromatiques à base d'un métal du groupe VIII. Ladite composition catalytique comprend soit du platine soit du platine/palladium, du rhénium et de l'étain, lesdits métaux étant supportés sur un support formé d'une zéolithe mordénite ou d'un système zéolithique formé d'une zéolithe mordénite et d'une zéolithe ZSM-5.

Le document WO 00/38834 divulgue un catalyseur pour la dismutation et la transalkylation de composés aromatiques. Ce catalyseur comprend un support formé d'une zéolithe mordénite et/ou beta, d'une zéolithe ZSM-5, d'une matrice inorganique, et des métaux déposés sur ledit support, Lesdits métaux sont le platine et soit le plomb soit l'étain.

Le document US 5.800.698 divulgue un système catalytique pour l'élimination de l'azote et/ou du soufre présents dans des charges hydrocarbonées. Ce système catalytique comprend une matrice, un support constitué de deux zéolithes différentes, par exemple une zéolithe ZSM-5 et une zéolithe beta, une première phase catalytiquement active comprenant deux métaux différents du groupe VIII, une deuxième phase catalytiquement active comprenant deux métaux différents groupe VIII et un métal du groupe VIB.

### Résumé

La présente invention concerne un catalyseur comportant au moins une zéolithe présentant des canaux dont l'ouverture est définie par un anneau à 10 atomes d'oxygène (10 MR), au moins une zéolithe présentant au moins des canaux ou des poches latérales dont l'ouverture est définie par un anneau à 12 atomes d'oxygène (12 MR), du gallium et du rhénium et au moins une matrice minérale poreuse. Le catalyseur selon la présente invention comporte en outre éventuellement au moins un métal choisi dans le groupe constitué par les métaux des groupes IVA et VIB. Chacune des zéolithes comprises dans le catalyseur selon l'invention contient du silicium et au moins un élément T choisi dans le groupe formé par l'aluminium, le fer, le gallium et le bore. Le catalyseur selon la présente invention est dépourvu de tout métal du groupe VIII.
La présente invention concerne également l'utilisation dudit catalyseur dans un procédé de transalkylation d'hydrocarbures alkylaromatiques tels que le toluène et les alkylaromatiques ayant au moins 9 atomes de carbone ou le benzène et les alkylaromatiques ayant au moins 9 atomes de carbone. Ce catalyseur est, en particulier, très performant dans le traitement de charges aromatiques C9+ contenant un pourcentage de molécules aromatiques ayant au moins 10 atomes de carbone et plus supérieur à 5 % poids, cette charge pouvant également contenir du benzène.

### Intérêt

Il a été découvert, de manière surprenante, qu'un catalyseur composite comprenant l'association d'au moins une zéolithe présentant des canaux dont l'ouverture est définie par un anneau à 10 atomes d'oxygène (10 MR) et d'au moins une zéolithe présentant au moins des canaux ou des poches latérales dont l'ouverture est définie par un anneau à 12 atomes d'oxygène (12 MR) ainsi que du gallium et du rhénium conduit à des performances catalytiques améliorées, notamment en termes d'activité, de stabilité et de sélectivité, dans les réactions de dismutation d'hydrocarbures alkylaromatiques tel que le toluène ou le benzène et/ou dans les réactions de transalkylation d'hydrocarbures alkylaromatiques tels que le toluène, le benzène et les triméthylbenzènes ou alkylaromatiques à plus de 9 atomes de carbone (C9+). Le catalyseur selon l'invention est, en particulier, très performant dans le traitement de charges aromatiques C9+ contenant un pourcentage élevé (supérieur à 5 % poids) de molécules aromatiques à 10 atomes de carbone et plus ce qui permet de valoriser ces molécules lourdes, par exemple les diméthyléthylbenzènes, les diéthylbenzènes, ayant généralement des groupements alkyles à plus d'un atome de carbone (éthyl, propyl...) en xylènes. Lorsqu'il est utilisé dans un procédé de transalkylation du benzène ou du toluène et de composés alkylaromatiques, le catalyseur selon l'invention conduit à une amélioration notable des rendements en benzène et en xylène obtenus, produits à haute valeur ajoutée, par rapport aux rendements obtenus avec des catalyseurs connus dans l'art antérieur.
De plus, en ajustant la quantité relative des deux zéolithes, celle à 10 MR et celle à au moins 12 MR, dans le catalyseur selon l'invention, il est possible de traiter une très large gamme de mélanges de charges hydrocarbonées.

### Description

La présente invention a pour objet un catalyseur comportant au moins une zéolithe présentant des canaux dont l'ouverture est définie par un anneau à 10 atomes d'oxygène (10 MR), au moins une zéolithe présentant au moins des canaux ou des poches latérales dont l'ouverture est définie par un anneau à 12 atomes d'oxygène (12 MR), du gallium et du rhénium et au moins une matrice minérale poreuse. Le catalyseur selon la présente invention est dépourvu de tout métal du groupe VIII.

Les zéolithes sont définies dans la classification "Atlas of Zeolite Structure Types", W. M Meier, D. H. Olson and Ch. Baerlocher, 5th revised edition, 2001, Elsevier auquel se réfère également la présente demande. Les zéolithes y sont classées selon la taille de leurs ouvertures de pores ou canaux. Conformément à l'invention, au moins une zéolithe comprise dans le catalyseur selon l'invention présentent des pores ou canaux dont l'ouverture est définie par un anneau à 10 atomes d'oxygène (ouverture à 10MR) et au moins une autre zéolithe comprise dans le catalyseur selon l'invention présentent au moins des pores ou des canaux ou des poches latérales dont l'ouverture est définie par un anneau à 12 atomes d'oxygène (ouverture à au moins 12MR). Selon l'invention, les canaux de la zéolithe ayant une ouverture à 10 MR appelée dans la suite de la description par zéolithe 10 MR sont des canaux principaux qui débouchent directement sur l'extérieur de ladite zéolithe. La zéolithe ayant une ouverture à au moins 12 MR appelée dans la suite de la description par zéolithe à au moins 12 MR présente au moins soit des canaux principaux 12 MR débouchant directement sur l'extérieur de ladite zéolithe soit des canaux secondaires 12 MR accessibles uniquement par des canaux principaux ayant une ouverture autre que 12 MR soit encore des poches latérales ("side pockets") dont l'ouverture est définie par un anneau à 12 atomes d'oxygène. Les zéolithes 10MR et au moins 12MR présentes dans le catalyseur selon l'invention comprennent du silicium et au moins un élément T choisi dans le groupe formé par l'aluminium, le fer, le gallium et le bore, de préférence l'aluminium. Elles sont, de préférence, pratiquement totalement, sous forme acide.

La zéolithe 10 MR présente dans le catalyseur selon l'invention est caractérisée par un rapport Si/Al compris entre 2 et 250, de préférence compris entre 5 et 150 et de manière très préférée compris entre 10 et 80. La teneur en sodium est inférieure à 0,2 % poids, de préférence inférieure à 0,1 % poids et de manière très préférée inférieure à 0,05 % poids par rapport au poids total de zéolithe sèche. Les zéolithes 10 MR préférées sont choisies parmi les zéolithes ZSM-5, IM-5 et ZSM-22. Ces zéolithes et leur mode de préparation sont bien connus de l'Homme du métier.

La zéolithe à au moins 12MR présente dans le catalyseur selon l'invention est caractérisée par un rapport Si/Al compris entre 2 et 250, de préférence compris entre 5 et 150 et de manière très préférée compris entre 10 et 80. La teneur en sodium est inférieure à 0,2 % poids, de préférence inférieure à 0,1 % poids et de manière très préférée inférieure à 0,05% poids par rapport au poids total de zéolithe sèche. Les zéolithes à au moins 12 MR, préférées dans le cadre de la présente invention, sont choisies parmi les zéolithes Béta, Y, mordénite, NU-87, ITQ-23, EU-1 et la boggsite. La zéolithe NU-87, de type structural NES possède des canaux principaux à 10MR et également des canaux secondaires à 12MR accessibles par les canaux à 10MR, telle que décrit dans le livre "Synthesis of microporous materials", Vol1, Ed M.L.Occelli and H.E.Robson, chap 24 (Casci J.L et al). La boggsite possède des canaux principaux à 10MR et 12 MR. La zéolithe EU-1 possède des canaux principaux à 10 MR et des poches latérales ("side pockets") à 12 MR. Ces zéolithes et leur mode de préparation sont bien connus de l'Homme du métier.

Les rapports Si/Al des zéolithes décrites ci-dessus sont ceux obtenus à l'issue de la synthèse desdites zéolithes ou bien obtenus après des traitements de désalumination post-synthèse bien connus de l'homme du métier, tels que et à titre non exhaustif les traitements hydrothermiques suivis ou non d'attaques acides ou bien encore les attaques acides directes par des solutions d'acides minéraux ou organiques.
Le rapport Si/Al global des zéolithes 10 MR et au moins 12 MR entrant dans la composition du catalyseur selon l'invention ainsi que la composition chimique des échantillons sont déterminés par fluorescence X et absorption atomique.

Les zéolithes 10 MR et au moins 12MR entrant dans la composition du catalyseur selon l'invention peuvent être calcinées et échangées par au moins un traitement par une solution d'au moins un sel d'ammonium de manière à obtenir la forme ammonium des zéolithes qui une fois calcinée conduisent à la forme hydrogène des dites zéolithes.

Les zéolithes 10 MR et au moins 12 MR entrant dans la composition du catalyseur selon l'invention sont au moins en partie, de préférence pratiquement totalement, sous forme acide, c'est-à-dire sous forme hydrogène (H⁺). Le rapport atomique Na/T est généralement inférieur à 10% et de préférence inférieur à 5% et de manière encore plus préférée inférieur à 1%.

Ledit catalyseur comprend en outre du gallium et du rhénium de préférence à une teneur comprise entre 0,01 et 5 % poids par rapport au poids total du catalyseur. Le catalyseur selon l'invention comporte éventuellement en outre au moins un métal choisi dans le groupe constitué par les métaux des groupes IVA et VIB, de préférence à une teneur comprise entre 0,01 et 5 % et de préférence entre 0,5 et 3 % poids par rapport au poids total du catalyseur. Parmi les métaux du groupe IVA, l'étain est préféré. Parmi les métaux du groupe VIB, le molybdène est préféré. Le catalyseur selon la présente invention est dépourvu de tout métal du groupe VIII.

La matrice minérale poreuse, présente à une teneur comprise entre 5 et 95 %, de préférence entre 10 et 90 %, de manière plus préférée entre 15 et 85% et de manière encore plus préférée entre 20 et 80% poids par rapport au poids total de catalyseur, est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium, les silice-alumines et le charbon, de préférence parmi les éléments du groupe formé par les alumines et les argiles, de manière encore plus préférée parmi les alumines.

Les zéolithes 10 MR et au moins 12MR présentes dans le catalyseur selon l'invention sont répertoriées dans l'atlas des Zéolithes et sont synthétisées selon les méthodes décrites dans les références citées dans cet ouvrage ("Atlas of Zeolite Structure Types", W. M Meier, D. H. Olson and Ch. Baerlocher, 5th revised edition, 2001) ou par toute autre méthode décrite dans la littérature ouverte à l'Homme du métier. Toute zéolithe commerciale peut par ailleurs être utilisée pour obtenir le catalyseur de l'invention.

Selon une première variante de préparation du catalyseur de l'invention, préalablement à leur mise en forme, au moins une des zéolithes précédemment décrites et comprise dans ledit catalyseur est soumise au dépôt d'un métal choisi parmi le rhénium et le gallium. De préférence, au moins la zéolithe 10 MR est soumise au dépôt d'un métal choisi parmi le rhénium et le gallium. Il est aussi possible que la zéolithe 10 MR soit soumise au dépôt d'un métal choisi parmi le rhénium et le gallium et que la zéolithe à au moins 12 MR soit soumise au dépôt d'un autre métal choisi parmi le rhénium et le gallium. Avantageusement, lorsque le catalyseur selon l'invention comporte du rhénium et du gallium et au moins un métal choisi dans le groupe constitué par les métaux des groupes IVA et VIB, la zéolithe 10 MR est soumise au dépôt du gallium et du rhénium et la zéolithe à au moins 12 MR est soumise au dépôt du métal choisi dans le groupe constitué par les métaux des groupes IVA et VIB. Les zéolithes ainsi chargées en métaux sont mélangées. Le mélange de ces zéolithes qui sont alors à l'état de poudre est réalisé par toutes les techniques de mélange de poudres connues de l'homme du métier.
Une fois le mélange des poudres de zéolithes, chargées en métaux, réalisé, le mélange est mis en forme par toute technique connue de l'homme de l'art. Il peut en particulier être mélangé à une matrice minérale poreuse, généralement amorphe, par exemple à une poudre humide de gel d'alumine. Le mélange est ensuite mis en forme, par exemple par extrusion au travers d'une filière.
La mise en forme peut être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ces formes connues de l'Homme du métier, et de manière encore plus préférée les alumines, par exemple l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées.

Après l'étape de mise en forme, le produit obtenu est soumis à une étape de séchage puis à une étape de calcination.

Selon une deuxième variante de préparation du catalyseur selon l'invention, un métal choisi parmi le gallium et le rhénium et éventuellement au moins un métal choisi dans le groupe constitué par les métaux des groupes IVA et VIB est(sont) déposé(s) sur le support après la mise en forme des zéolithes exemptes de métaux, par tout procédé connu de l'homme du métier et permettant le dépôt du métal sur zéolithes. On désigne par le terme "support", le mélange de zéolithes (exemptes de métaux) avec au moins une matrice minérale poreuse après mise en forme, séchage et calcination.
Le support du catalyseur de la présente invention renferme généralement les teneurs suivantes en matrice et zéolithes :
- 5 à 95% poids, de préférence 10 à 90% poids, de manière plus préférée de 15 à 85% poids et de manière très préférée de 20 à 80% poids de zéolithes telles que au moins une zéolithe soit choisie parmi les zéolithes 10MR et au moins une zéolithe soit choisie parmi les zéolithes à au moins 12MR,
- 5 à 95%, de préférence de 10 à 90%, de manière plus préférée de 15 à 85% et de manière très préférée de 20 à 80% poids d'au moins une matrice minérale poreuse amorphe ou mal cristallisée de type oxyde.

Pour le dépôt de métal sur les zéolithes selon la première et la deuxième variante de préparation du catalyseur selon l'invention, on peut utiliser la technique d'échange cationique avec compétition où le compétiteur est de préférence le nitrate d'ammonium, le rapport de compétition étant au moins égal à environ 20 et avantageusement d'environ 30 à 200. On peut utiliser aussi la technique d'imprégnation à sec ou de coprécipitation.
Les sources de rhénium qui peuvent être utilisées sont également bien connues de l'Homme du métier. On utilise habituellement un complexe de perrhénate d'ammonium (NH₄)ReO₄ ou l'acide perrhénique. Les sources de gallium qui peuvent être utilisées sont également bien connues de l'Homme du métier. Le nitrate de gallium Ga(NO₃)₃ est préféré. Les sources des métaux du groupe VIB qui peuvent être utilisées sont également bien connues de l'Homme du métier. Dans le cas du molybdène, on peut utiliser les acides molybdiques et leurs sels en particulier les sels d'ammonium tels que le molybdate d'ammonium, l'heptamolybdate d'ammonium ainsi que l'acide phosphomolybdique. De préférence, on utilise l'heptamolybdate d'ammonium (NH₄)₆Mo₇O₂₄. Le dépôt du gallium et du rhénium et éventuellement des groupes IVA et VIB est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures, de préférence on opérera entre 350° et 550°C pendant 2 à 5 heures.

On peut également déposer le métal non plus directement sur les zéolithes, mais sur la matrice minérale poreuse (par exemple le liant aluminique) du support, avant ou après l'étape de mise en forme, en mettant en oeuvre un échange anionique. On peut citer par exemple dans le cas du dépôt de Rhénium l'utilisation de l'acide perrhénique HReO₄ pour le Rhénium. En général après le dépôt de métal, le catalyseur est comme précédemment soumis à une calcination puis réduit sous hydrogène comme indiqué ci-dessus.

Dans une autre variante de l'invention, chaque zéolithe est mise en forme indépendamment avec un liant. Le mélange des zéolithes peut être réalisés après mise en forme (extrudés ou grains). Les métaux sont déposés avant ou après mélange des zéolithes mis en forme, de préférence avant. Un métal différent peut ainsi être déposé sur les deux zéolithes mises en forme. De préférence, le molybdène est déposé sur une des deux zéolithes, de préférence il est déposé sur la zéolithe à au moins 12 MR.

Dans le cas où le catalyseur contient plusieurs métaux, ces derniers peuvent être introduits soit tous de la même façon soit par des techniques différentes, avant ou après mise en forme selon la variante de préparation du catalyseur employée et dans n'importe quel ordre. Dans le cas où la technique utilisée est celle de l'échange ionique, plusieurs échanges successifs peuvent être nécessaires pour introduire les quantités requises de métaux.

Quelle que soit la variante de préparation du catalyseur selon l'invention, on peut procéder après la calcination dudit catalyseur à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu ex situ ou in situ, par rapport au lieu d'utilisation dudit catalyseur dans une réaction donnée.

La répartition entre les deux zéolithes de chacun des groupes définis précédemment est telle que la teneur en zéolithe(s) choisie(s) dans le groupe formé par les zéolithes 10MR peut varier de 1 % à 99%, de préférence de 5 à 95% et de manière encore plus préférée de 10 à 90% en pourcentages relatifs de l'ensemble des zéolithes introduites dans le catalyseur. De même, la teneur en zéolithe à au moins 12MR varie de 1% à 99%, de préférence de 5 à 95% et de manière encore plus préférée de 10 à 90%, en pourcentages relatifs, de l'ensemble des zéolithes introduites dans le catalyseur.

Le catalyseur selon la présente invention est mis en forme sous la forme de grains de différentes formes et dimensions. Il est utilisé en général sous la forme d'extrudés cylindriques ou polylobés tels que bilobés, trilobés, polylobés de forme droite ou torsadée, mais peut éventuellement être fabriqué et employé sous la forme de poudre concassées, de tablettes, d'anneaux, de billes, de roues. De manière très préférée, le catalyseur selon l'invention comporte du gallium et du rhénium sur la zéolithe 10 MR.

Le catalyseur de la présente invention peut éventuellement contenir du soufre. Dans ce cas, le soufre est introduit sur le catalyseur mis en forme, calciné, contenant le ou les élément(s) cité(s) précédemment, soit *in situ* avant la réaction catalytique, soit *ex situ*. La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction. Dans le cas d'une sulfuration *in situ*, la réduction, si le catalyseur n'a pas été préalablement réduit, intervient avant la sulfuration. Dans le cas d'une sulfuration *ex situ*, on effectue la réduction puis la sulfuration.

Le catalyseur selon l'invention est utilisé pour la conversion d'hydrocarbures.

L'invention concerne en particulier l'utilisation dudit catalyseur dans un procédé de transalkylation d'une charge d'hydrocarbures alkylaromatiques, et de préférence de transalkylation du benzène ou du toluène et d'hydrocarbures alkylaromatiques généralement en C₉⁺ (c'est à dire à au moins 9 atomes de carbone par molécule), avec des mélanges benzène-AC9+ ou toluène - AC₉⁺ (où AC₉⁺ désigne les hydrocarbures alkylaromatiques comportant au moins 9 atomes de carbone par molécule) pouvant contenir de 1 à 100% de AC₉⁺ par rapport au mélange total. Ledit catalyseur se révèle très efficace pour ladite utilisation, car il se révèle être particulièrement actif, sélectif et stable, même en présence de charges à traiter contenant une grande quantité d'aromatiques lourds AC₉⁺, ces aromatiques lourds pouvant contenir une grande proportion d'AC₁₀⁺. Ainsi, des charges AC₉⁺ contenant au moins 5 % et jusqu'à 25 % poids, et même davantage d'AC₁₀⁺ peuvent être valorisées. A titre d'exemples, on peut citer de manière non exhaustive, les diméthyléthylbenzènes, les diéthylbenzènes, les propyléthylbenzènes... L'utilisation de ce catalyseur en transalkylation d'alkylaromatiques lourds est donc particulièrement intéressante.

Les conditions opératoires pour ladite utilisation sont généralement les suivantes : une température comprise entre 250 et 650°C et de préférence entre 350 et 550°C ; une pression comprise entre 1 et 6 MPa et de préférence entre 2 et 4,5 MPa ; une vitesse spatiale d'alimentation, exprimée en kilogramme de charge introduite par kilogramme de catalyseur et par heure, comprise entre 0,1 et 10 h⁻¹ et de préférence entre 0,5 et 4 h⁻¹ ; un rapport molaire hydrogène sur hydrocarbures compris entre 2 et 20 et de préférence entre 3 et 12 mol/mol.

Les exemples qui suivent illustrent l'invention sans pour autant en limiter la portée.

### Exemple 1 : Préparation de catalyseurs à base de zéolithes 10MR, à base de zéolithes à au moins 12MR et à base d'une zéolithe 10MR et d'une zéolithe à au moins 12MR.

Les zéolithes utilisées pour préparer les catalyseurs exemplifiant l'invention sont présentées dans le tableau n°1 avec leur composition (rapport atomique Si / Al) et leur teneur résiduelle en sodium. Les cinq zéolithes concernées sont sous forme acide.
Les zéolithes Béta, Mordénite et ZSM-5 sont des zéolithes commerciaies (PQ).
La zéolithe NU-87 a été synthétisée d'après la demande de brevet européen EP-A-0.377.291 ou le brevet EP-B-0.378.916. Elle possède un rapport atomique Si/Al global égal à 17,2, une teneur pondérale en sodium égale à 1256 ppm. Cette zéolithe NU-87 subit, tout d'abord, une calcination dite sèche à 550°C sous flux d'air et d'azote durant 6 heures. Puis, le solide obtenu est soumis à un échange ionique dans une solution de NH₄NO₃ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires.
A l'issue de ces traitements, la zéolithe obtenue se trouve sous sa forme H et possède un rapport Si/Al atomique global égal à 33,3, et une teneur en Na de 10 ppm.
La zéolithe IM-5 est synthétisée selon l'exemple n°1 de la demande de brevet FR-A-2754809 ou du brevet US 6.136.290.

**Tableau n°1: zéolithes à 10MR et au moins 12 MR**

| zéolithes | Si/Al (FX) | Na (ppm) | Type |
|---|---|---|---|
| Béta | 12.5 | 87 | 12 MR |
| ZSM-5 | 17.5 | 132 | 10 MR |
| MOR | 10 | 109 | 12 MR |
| IM-5 | 12 | 84 | 10 MR |
| NU-87 | 33.3 | 10 | 10 & 12MR |

Les zéolithes sont ensuite mises en forme par extrusion avec un gel d'alumine de manière à obtenir, après séchage et calcination sous air sec, un support qui contient en poids 80 % de zéolithe et 20 % d'alumine. La partie zéolithique du support est constituée d'une zéolithe seule ou d'un mélange mécanique de deux zéolithes différentes, réalisé avant la mise en forme.

Pour la préparation des catalyseurs A à N, le support zéolithique comprenant une zéolithe ou un mélange de deux zéolithes différentes est soumis à une imprégnation à sec par une solution de précurseur métallique (perrhénate d'ammonium pour le rhénium, heptamolybdate d'ammonium pour le molybdène, nitrate de gallium pour le gallium, de manière à déposer un pourcentage de métal visé. Le solide humide est ensuite séché à 120°C pendant 12 heures et calciné sous un débit d'air sec à la température de 500°C pendant une heure. La composition des catalyseurs obtenus est reportée dans le tableau n°2.
Pour les catalyseurs O et P, le support extrudé contenant la zéolithe Béta soumis au dépôt de molybdène (catalyseur M) ou de gallium (catalyseur N) est mélangé mécaniquement au support extrudé contenant la zéolithe ZSM-5 soumis au dépôt de rhénium (catalyseur B).

**Tableau n°2 : catalyseurs contenant soit une zéolithe 10 MR soit une zéolithe à au moins 12 MR soit une zéolithe 10 MR et une zéolithe à au moins 12 MR**

| Catalyseur | %Al2O3 | zéolithe(s) | Zéolithes ratio | % Métal |
|---|---|---|---|---|
| A | 20 | béta | 100 | 0.5% Re |
| B | 20 | ZSM-5 | 100 | 0.5% Re |
| C | 20 | MOR | 100 | 0.5% Re |
| D | 20 | IM-5 | 100 | 0.25% Re |
| E | 20 | NU-87 | 100 | 0.25% Re |
| F | 20 | béta + ZSM-5 | 50 / 50 | 0.5% Re |
| G | 20 | MOR + ZSM-5 | 50 / 50 | 0.5% Re |
| H | 20 | NU-87 + ZSM-5 | 50 / 50 | 0.25% Re |
| I | 20 | béta + IM-5 | 50 / 50 | 0.25% Re |
| J | 20 | NU-87 + IM-5 | 34 / 66 | 0.25% Re |
| K | 20 | NU-87 + IM-5 | 66 / 34 | 0.25% Re |
| M | 20 | béta | 100 | 1 % Mo |
| N | 20 | béta | 100 | 1 % Ga |
| O | 20 | béta + ZSM-5 | 50 / 50 | 0.5% Mo / béta 0.25%Re/ZSM-5 |
| P | 20 | béta + ZSM-5 | 50/50 | 0.5% Ga / béta 0.25%Re ZSM-5 |

Les catalyseurs A à O sont comparatifs. Le catalyseur P est conforme à l'invention.

### Exemple 2 : Performances catalytiques de catalyseurs à base de zéolithes 10MR à base de zéolithes à au moins 12MR et à base d'une zéolithe 10MR et d'une zéolithe à au moins 12MR

Les catalyseurs sont préalablement réduits sous hydrogène à 450°C pendant 2h.
Les tests catalytiques sont réalisés dans les conditions opératoires suivantes:
- température : 400°C
- pression totale : 25 bar
- H2/HC= 8.5 mol/mol
- WHSV = 4h⁻¹ (masse de charge par gramme de catalyseur et par heure)

La charge est constituée de 50% de toluène, 16% d'éthyltoluène, 28% de triméthylbenzène et 6% d'aromatiques à au moins 10 atomes de carbone.

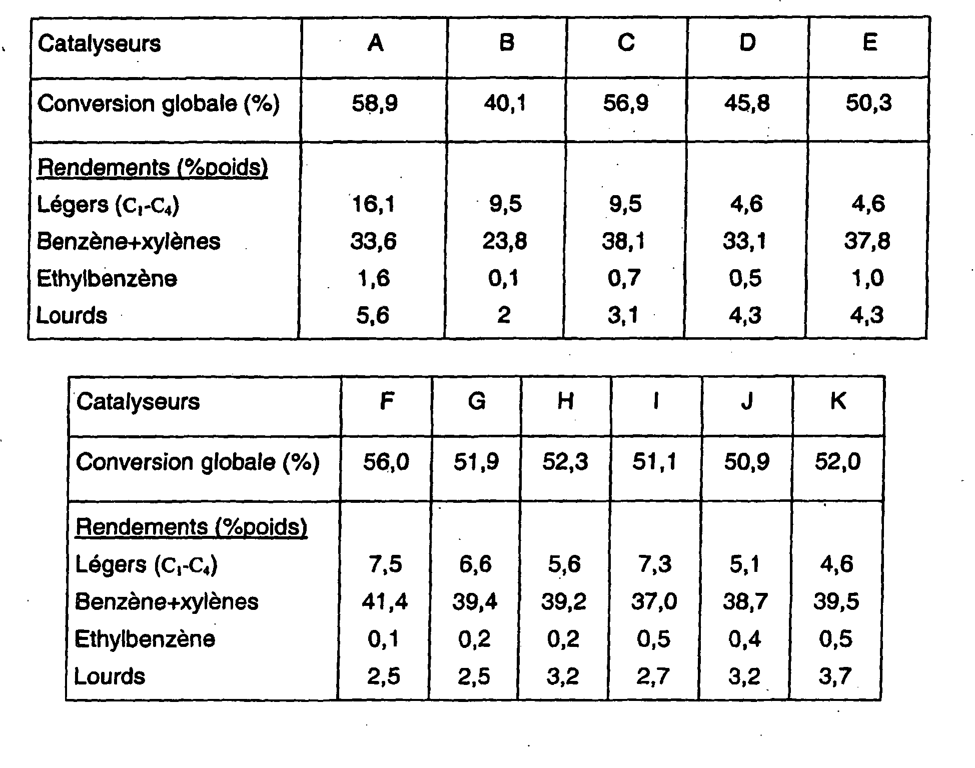

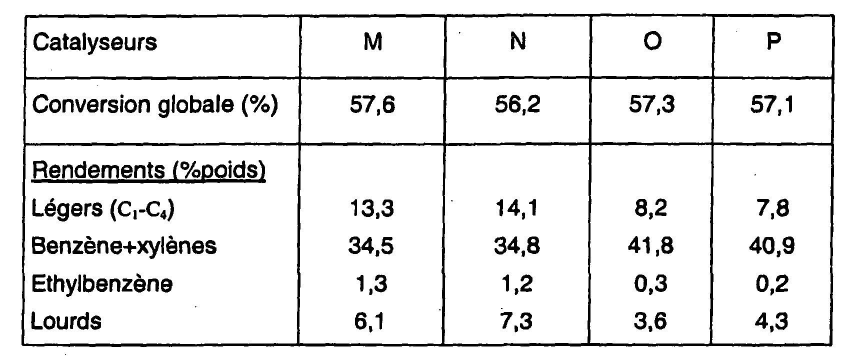

L'utilisation de catalyseurs contenant des mélanges constitués d'au moins une zéolithe 10 MR, d'au moins une zéolithe à au moins 12 MR, au sens de la présente invention de gallium et de rhénium permet d'augmenter le rendement en xylènes et benzène qui sont les produits cibles de la réaction de transalkylation car hautement valorisables.

## Revendications

1. Catalyseur comportant au moins une zéolithe présentant des canaux dont l'ouverture est définie par un anneau à 10 atomes d'oxygène (10 MR)et choisie parmi les zéolithes ZSM-5, IM-5 et ZSM-22, au moins une zéolithe présentant au moins des canaux ou des poches latérales dont l'ouverture est définie par un anneau à 12 atomes d'oxygène (12 MR) et choisie parmi les zéolithes beta, Y, mordénite, NU-87 EU-1, ITQ-23 et boggsite, du gallium et du rhénium au moins une matrice minérale poreuse, ledit catalyseur étant dépourvu de tout métal du groupe VIII.

2. Catalyseur selon la revendication 1 comportant au moins un métal choisi dans le groupe constitué par les métaux des groupes IVA et VIB.

3. Catalyseur selon l'une des revendications 1 à 2 dans lequel le métal du groupe VIB est le molybdène.

4. Catalyseur selon l'une des revendications 1 à 3 comprenant du soufre.

5. Utilisation du catalyseur selon l'une des revendications 1 à 4 dans un procédé de transalkylation d'une charge d'hydrocarbures alkylaromatiques.

6. Utilisation selon la revendication 5 dans un procédé de transalkylation du benzène ou du toluène et d'hydrocarbures alkylaromatiques ayant au moins 9 atomes de carbone par molécule.

7. Utilisation selon la revendication 6 pour le traitement de charges aromatiques contenant au moins 5% poids d'aromatiques ayant au moins 10 atomes de carbone.

8. Utilisation selon l'une des revendications 5 à 7 à une température comprise entre 250 et 650°C, une pression comprise entre 1 et 6 MPa, une vitesse spatiale d'alimentation, exprimée en kilogramme de charge introduite par kilogramme de catalyseur et par heure, comprise entre 0,1 et 10 h⁻¹, un rapport molaire hydrogène sur hydrocarbures compris entre 2 et 20.

## Claims

1. A catalyst comprising at least one zeolite with channels with openings defined by a ring having 10 oxygen atoms (10 MR) and selected from ZSM-5, IM-5 and ZSM-22 zeolites, at least one zeolite with at least channels or side pockets with openings defined by a ring having 12 oxygen atoms (12 MR) and selected from beta, Y, mordenite, NU-87, EU-1, ITQ-23 and boggsite zeolites, gallium and rhenium, at least one porous mineral matrix, said catalyst being free from any group VIII metal.

2. A catalyst according to claim 1, comprising at least one metal selected from the group constituted by group IVA and VIB metals.

3. A catalyst according to claim 1 or claim 2, in which the group VIB metal is molybdenum..

4. A catalyst according to one of claims 1 to 3, comprising sulphur.

5. Use of a catalyst according to one of claims 1 to 4, in a process for the transalkylation of an alkylaromatic hydrocarbon feed.

6. Use according to claim 5, in a process for the transalkylation of benzene or toluene and alkylaromatic hydrocarbons containing at least 9 carbon atoms per molecule..

7. Use according to claim 6, for the treatment of an aromatic feed containing at least 5% by weight of aromatics containing at least 10 carbon atoms.

8. Use according to one of claims 5 to 7, at a temperature in the range 250°C to 650°C, a pressure in the range 1 to 6 MPa, a supply space velocity, expressed in kilograms of feed introduced per kilogram of catalyst per hour, in the range 0.1 to 10 h⁻¹, and a mole ratio of hydrogen to hydrocarbons in the range 2 to 20..

## Patentansprüche

1. Katalysator, umfassend mindestens einen Zeolithen, der Kanäle aufweist, deren Öffnung durch einen Ring mit 10 Sauerstoffatomen definiert ist (10 MR) und der aus den Zeolithen ZSM-6, IM-5 und ZSM-22 ausgewählt ist, mindestens einen Zeolithen, der mindestens Kanäle oder seitliche Taschen aufweist, deren Öffnung durch einen Ring mit 12 Sauerstoffatomen definiert ist (12 MR) und der aus den Zeolithen beta, Y, Mordenit, NU-87, EU-1, ITQ-23 und Boggsit, Gallium und Rhenium ausgewählt ist, mindestens eine poröse Mineralmatrix, wobei der Katalysator frei von jeglichem Metall der Gruppe VIII ist.

2. Katalysator nach Anspruch 1, der mindestens ein Metall umfasst, das ausgewählt ist aus der Gruppe bestehend aus den Metallen der Gruppen IVA und VIB.

3. Katalysator nach einem der Ansprüche 1 bis 2, wobei das Metall der Gruppe VIIB Molybdän ist.

4. Katalysator nach einem der Ansprüche 1 bis 3, der Schwefel umfasst.

5. Verwendung des Katalysators nach einem der Ansprüche 1 bis 4 in einem Verfahren zur Transalkylierung einer Beschickung aus alkylaromatischen Kohlenwasserstoffen.

6. Verwendung nach Anspruch 5 in einem Verfahren zur Transalkylierung von Benzen oder Toluen und alkylaromatischen Kohlenwasserstoffen, die mindestens 9 Kohlenstoffatome je Molekül haben.

7. Verwendung nach Anspruch 6 zur Behandlung aromatischer Beschickungen, die mindestens 5 Gew.-% aromatische Verbindungen enthalten, die mindestens 10 Kohlenstoffatome haben.

8. Verwendung nach einem der Ansprüche 5 bis 7 bei einer Temperatur im Bereich zwischen 250 und 650 °C, einem Druck im Bereich zwischen 1 und 6 MPa, einer Versorgungsraumgeschwindigkeit, ausgedrückt in Kilogramm eingeführter Beschickung pro Kilogramm Katalysator und pro Stunde, im Bereich zwischen 0,1 und 10 h⁻¹, einem Molverhältnis von Wasserstoff zu Kohlenwasserstoffen im Bereich zwischen 2 und 20.
